# EUROPEAN PATENT APPLICATION

(11) **EP 4 406 563 A1**
(43) Date of publication of application: **31.07.2024**
(21) Application number: 21958116.2
(22) Date of filing: 20.10.2021
(51) Int. Cl.: A61L 27/36, C07K 19/00, A61K 9/133, A61P 19/00

(54) **MODIFIED SIS MEMBRANE FOR TISSUE REPAIR, PREPARATION METHOD THEREFOR AND USE THEREOF**

(30) Priority: 24.09.2021 CN 202111120787
(71) Applicant: Beijing Biosis Healing Biological Technology Co., Ltd., Beijing 102600 (CN)
(72) Inventor: ZHAO, Bo, Beijing 102600 (CN); WANG, Huasheng, Beijing 102600 (CN); WEI, Pengfei, Beijing 102600 (CN); JING, Wei, Beijing 102600 (CN); LIU, Zihao, Beijing 102600 (CN)
(74) Representative: Perani & Partners S.p.A.
(86) International application number: PCT/CN2021/124991
(87) International publication number: WO 2023/045001

(57) **Abstract**

The present disclosure relates a modified SIS membrane for tissue repair, a preparation method therefor and a use thereof. Specifically provided are a composition comprising a fusion polypeptide-modified SIS membrane and an extracellular vesicle, as well as a preparation method for and a use of the composition. It is proved that the fusion polypeptide-mediated SIS membrane and the composition containing the SIS membrane and the extracellular vesicle have excellent biological functions, and provide a broad prospect for treating bone defects or facilitating tissue healing or bone regeneration.

## Description

### TECHNICAL FIELD

The present disclosure relates to the field of biomedical materials, in particular to a modified SIS membrane for tissue repair, a preparation method therefor, and a use thereof.

### BACKGROUND

Tissue damage caused by disease or trauma causes pain, functional defects, and aesthetic and psychological problems in patients. Appropriate treatment promotes cell proliferation and migration by providing an environment conducive to wound healing, thus inducing tissue regeneration. Traditional patch materials, such as polypropylene (PP), polyethylene terephthalate (PET), and polyvinylidene fluoride (PVDF), are prone to cause chronic pain, severe infection and other complications because they are nondegradable and cannot provide a microenvironment suitable for tissue repair. To our knowledge, the local microenvironment around the wound, which involves cell-to-cell communication, cell-to-extracellular matrix interaction, signalling molecules, oxygen and nutrition, contributes to the course of tissue regeneration. Therefore, a novel biomimetic patch that can not only degrade but also establish a proper local microenvironment for tissue regeneration is urgently needed.

The small intestinal submucosa (SIS) membrane is one of the most widely applied extracellular matrix (ECM) materials in the treatment of cardiovascular disease, hernia repair, cervical surgery, tracheal tympanic membrane repair and other soft tissue damage because of its extensive source and great degradability. The natural composition of collagen and abundant bioactive factors, including transforming growth factor-β (TGF-β), glycoproteins and fibronectin, provide an SIS membrane with a three-dimensional spatial structure for cellular adhesion and spread as well as excellent biocompatibility.

In fact, membrane implantation relies mainly on the pattern of endogenous tissue regeneration to complete continuous and dynamic remodelling, which requires a variety of chemical agents and cytokines. Therefore, the presence of bioactive components in the microenvironment is crucial for this complicated process. Considering that biological patches are exogenous biomaterials, the repair process induced by SIS material is extremely dependent on the cellular state (such as recruitment and differentiation of stem cells) and the potential immunogenicity of the material itself, so the SIS membrane is still insufficient in microenvironment simulation and tissue induction. Therefore, loading more bioactive substances on SIS material is conducive to regenerative wound therapy.

Mesenchymal stem cells (MSCs), as seed cells effectively applied in tissue engineering, can increase cell-to-cell communication by regulating the expression of cytokines and growth factors. However, intra-arterial injection of MSCs has been shown to lead to myocardial microinfarction in dogs, unveiling the risk of immune rejection and embolism.

Extracellular vesicles derived from umbilical cord mesenchymal stem cells (ucMSCs) can regulate the cellular immunologic response, revascularization and tissue reconstruction during wound healing. Moreover, extracellular vesicle miR-1263 of ucMSCs-EVs directly targets Mob 1 in recipient cells to inhibit apoptosis and achieves rapid cell proliferation and finally obtains a better tissue-inductive property. However, traditional loading methods for EVs, such as direct absorption and chemical coating, have the disadvantages of low loading efficiency, sudden release *in vivo* and destruction of EVs' structure, which may affect the therapeutic effect and limit wide application.

Tissue injury, which often occurs in daily life, remains challenging in clinical medicine. Developing a novel biomaterial with the capability to provide an ideal microenvironment and homeostasis around the wound is highly desirable for actual tissue regenerative medicine.

### SUMMARY

### Problem to be solved

In view of the deficiencies of the prior art, the present disclosure provides a chimeric peptide-modified SIS membrane and a composition comprising the chimeric peptide-modified SIS membrane and an extracellular vesicle, which have a function of facilitating soft tissue healing and bone regeneration. The present disclosure verifies that the chimeric peptide-modified SIS membrane and the composition comprising the chimeric peptide-modified SIS membrane and an extracellular vesicle can be used for clinical treatment of bone defects.

### Solution to the problem

The present disclosure proposes the following technical solution.
(1) A composition, comprising a fusion polypeptide-modified SIS membrane and an extracellular vesicle, wherein a sequence of the fusion polypeptide comprises at least one selected from a group consisting of sequences as set forth in the following SEQ ID NOs:
   (i) a group consisting of a sequence as set forth in SEQ ID NO: 5, a sequence as set forth in SEQ ID NO: 6, a sequence as set forth in SEQ ID NO: 7, a sequence as set forth in SEQ ID NO: 8;
   (ii) a sequence having one, two, three, four, or five conservative substitutions as compared with the sequence as set forth in (i).
(2) The composition according to (1), wherein the sequence of the fusion polypeptide is composed of at least one selected from a group consisting of sequences as set forth in the following SEQ ID NOs:
   (i) a group consisting of a sequence as set forth in SEQ ID NO: 5, a sequence as set forth in SEQ ID NO: 6, a sequence as set forth in SEQ ID NO: 7, a sequence as set forth in SEQ ID NO: 8;
   (ii) a sequence having one, two, three, four, or five conservative substitutions as compared with the sequence as set forth in (i).

   In a specific embodiment, the fusion polypeptide has a sequence having one or two conservative substitutions as compared with the sequence as set forth in SEQ ID NO: 5, the sequence as set forth in SEQ ID NO: 6, the sequence as set forth in SEQ ID NO: 7, the sequence as set forth in SEQ ID NO: 8, and the composition comprising the fusion polypeptide-modified SIS membrane still has at least one use of the following (a) to (c):
   (a) as, or for preparation of, a biomaterial for bone generation; (b) as, or for preparation of, a biomaterial for facilitating tissue healing; (c) as, or for preparation of, a biomaterial for treating bone defects.
(3) The composition according to (1) or (2), wherein the sequence of the fusion polypeptide on the fusion polypeptide-modified SIS membrane is set forth in SEQ ID NO: 5 and SEQ ID NO: 6.
   In a specific embodiment, the fusion polypeptide has a sequence having one or two conservative substitutions as compared with the sequence as set forth in SEQ ID NO: 5 or the sequence as set forth in SEQ ID NO: 6, and the composition comprising the fusion polypeptide-modified SIS membrane still has at least one use of (a) to (c):
   (a) as, or for preparation of, a biomaterial for bone generation; (b) as, or for preparation of, a biomaterial for facilitating tissue healing; (c) as, or for preparation of, a biomaterial for treating bone defects.
(4) The composition according to any one of (1) to (3), wherein the extracellular vesicle derives from a mesenchymal stem cell; preferably, the extracellular vesicle is an extracellular vesicle derived from an umbilical cord mesenchymal stem cell.
(5) The composition according to any one of (1) to (4), wherein the composition is obtained by incubating the fusion polypeptide-modified SIS membrane and the extracellular vesicle.
(6) A preparation method for the composition according to any one of (1) to (5), wherein a method for the modification comprises the following steps (a) to (d) or (i) to (iv):
   (a) dissolving the fusion polypeptide in a solvent to obtain a solution containing fusion polypeptides;
   (b) mixing and incubating the solution obtained in step (a) and extracellular vesicles, to obtain an incubation solution;
   (c) applying the incubation solution obtained in step (b) to a surface of the SIS membrane; preferably, immersing the SIS membrane in the incubation solution obtained in step (b) ;
   (d) drying the SIS membrane having the incubation solution on the surface to obtain the composition; or
      (i) dissolving the fusion polypeptide in a solvent to obtain a solution containing fusion polypeptides;
      (ii) applying the solution obtained in step (i) to a surface of the SIS membrane; preferably, immersing the SIS membrane in the solution obtained in step (i) ;
      (iii) applying extracellular vesicles to a surface of the SIS membrane obtained in step (ii) ;
      (iv) drying the SIS membrane obtained from step (iii) to obtain the composition.
(7) The preparation method according to (6), wherein the extracellular vesicle is an extracellular vesicle derived from a mesenchymal stem cell; preferably, the extracellular vesicle is an extracellular vesicle derived from an umbilical cord mesenchymal stem cell.
(8) Use of the composition according to any one of (1) to (5) or the composition obtained by the preparation method according to (6) or (7) in at least one of the following (a) to (c):
   (a) as, or for preparation of, a biomaterial for bone generation;
   (b) as, or for preparation of, a biomaterial for facilitating tissue healing;
   (c) as, or for preparation of, a biomaterial for treating bone defects.
(9) A method for treating bone defects or for facilitating tissue healing or bone regeneration, comprising a step of administering the composition according to any one of (1) to (5) or the composition obtained by the preparation method according to (6) or (7) to a subject.

### Effect

Small intestinal submucosa (SIS) membranes have a precise spatial structure and good biocompatibility. Extracellular vesicles realize rapid cell proliferation and migration with almost no immunoreaction by creating a satisfying microenvironement.

In a specific embodiment, the extracellular vesicle is an extracellular vesicle derived from umbilical cord mesenchymal stem cell (ucMSCs).

In the present disclosure, the fusion polypeptide-mediated SIS membrane and the fusion peptide-mediated extracellular vesicle can modify the surface of the SIS membrane by a specific combination thereof. In vitro studies proved that the modified SIS membrane can facilitate cell migration and spreading. By constructing a rat abdominal wall defect model, it was further proved that the modified SIS membrane is more conducive to tissue regeneration.

In a specific embodiment, the present disclosure designed a series of fusion polypeptides to achieve specific combination and affinity loading of extracellular vesicles onto the SIS membrane by connecting LHERHLNNN and KELNLVY with CP05, with or without a flexible linker (GGGGS). This modification conferred a better tissue regeneration effect on the SIS membrane.

The present disclosure proved that the fusion polypeptide-mediated SIS membrane and the composition comprising the SIS membrane and extracellular vesicles have excellent biological functions, and provide a broad prospect for treating bone defects or facilitating tissue healing or bone regeneration.

### BRIEF DESCRIPTION OF THE DRAWINGS

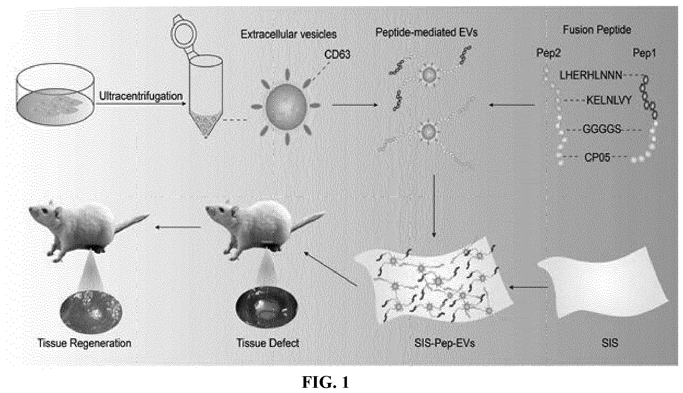

Fig. 1 is a schematic diagram of the SIS membrane modified by fusion peptide-mediated extracellular vesicles.

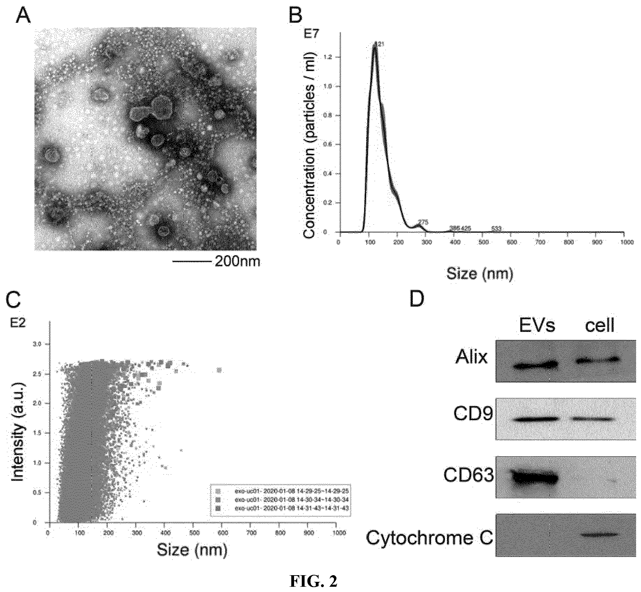

Fig. 2 shows the characterization of extracellular vesicles. A of Fig. 2 is a Transmission electron microscopy (TEM) image of extracellular vesicles (×20k); B of Fig. 2 shows particle size distribution of extracellular vesicles revealed by Nanosight; C of Fig. 2 shows intensity and size distribution of extracellular vesicles; D of Fig. 2 shows Western blot analysis to identify the expression of Alix, CD63, CD9 and Cytochrome C in EVs.

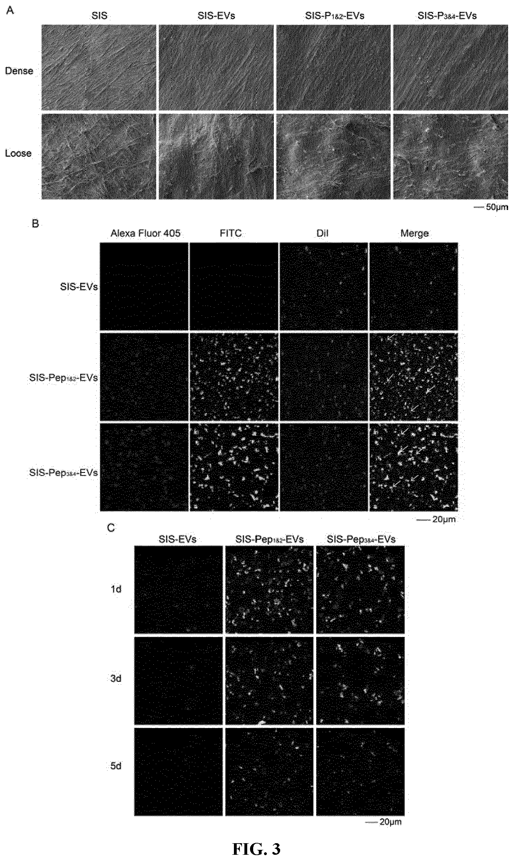

Fig. 3 shows the characterization of SIS, SIS-EVs, SIS-Pep1&2-EVs, and SIS-Pep3&4-EVs membranes. A of Fig. 3 shows SEM images for observation of the surface morphology, with the arrows referring to fusion polypeptides and extracellular vesicles (×500); B of Fig. 3 shows CLSM images of loose layers of the SIS-EVs group, the SIS-Pep1&2-EVs group, and the SIS-Pep3&4-EVs group, with Pep1 or Pep3 being labeled with AlexaFluor405, Pep2 or Pep4 labeled with FITC, and extracellular vesicles labeled with DiI, and the arrows referring to extracellular vesicles co-localized with fusion polypeptides; C of Fig. 3 shows release of fusion peptides and extracellular vesicles after immersion in PBS for 1, 3, and 5 days.

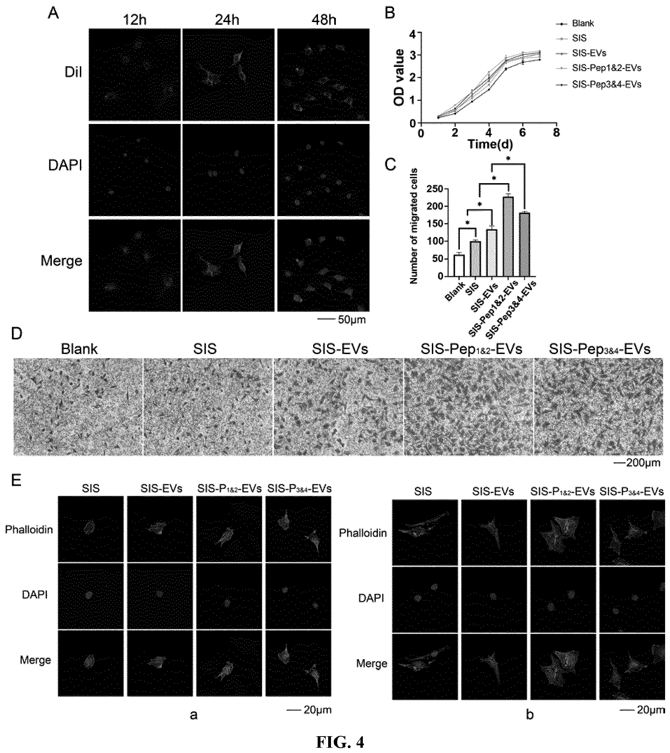

Fig. 4 shows cellular biological behaviors and biosafety evaluation results of SIS, SIS-Pep1&2-EVs, and SIS-Pep3&4-EVs groups. A of Fig. 4 shows CLSM of extracellular vesicles taken up by cells at 12 hours, 24 hours, and 48 hours. B of Fig. 4 shows CCK-8 growth curves of NIH3T3 cells seeded on each sample after culture for 1, 2, 3, 4, 5, 6, and 7 days. C of Fig. 4 shows a quantitative analysis of cell migration. One-way ANOVA was used for statistical analysis. Data is expressed as mean ± SD, n=3, *P<0.05. D of Fig. 4 shows Transwell images for evaluating the migration of NIH3T3 cells cultured in the 5 groups. E of Fig. 4 shows fluorescent staining after a) 6 and b) 24 hours of cell culture showing the morphological characteristics of cells.

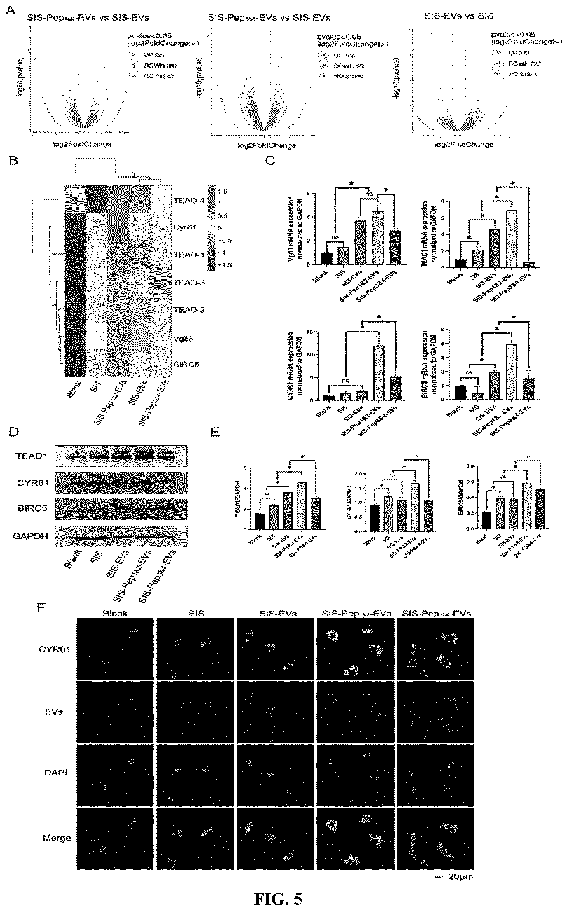

Fig. 5 shows research results of the mechanism for promoting tissue regeneration. A of Fig. 5 shows differentially expressed genes screened by RNAseq analysis in each group. B of Fig. 5 shows a heat map of cluster analysis of related genes. C of Fig. 5 shows qRT-PCR analysis of Vgll3, TEAD1, CYR61, and BIRC5 expression level in each group at day 3. One-way ANOVA was used for statistical analysis. Data is expressed as average value±SD, n=3, *P<0.05. D of Fig. 5 shows the western bolt for TEAD1, CYR61, and BIRC5 in NIH3T3 cells growing in different groups at day 3. GAPDH was used as a standardized control. E of Fig. 5 shows a quantitative analysis of the western blotting analysis. One-way ANOVA was used for statistical analysis. Data is expressed as average value±SD, n=3, *P<0.05. F of Fig. 5 shows the immunofluorescence image of CYR61.

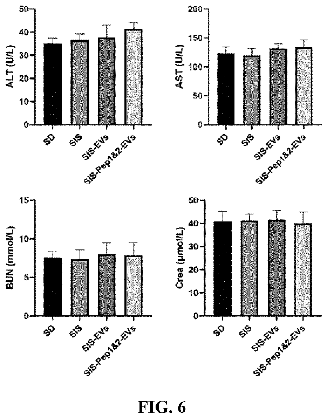

Fig. 6 shows the serum analysis result of ALT, AST, BUN, and Crea for each group of the modified SIS membrane.

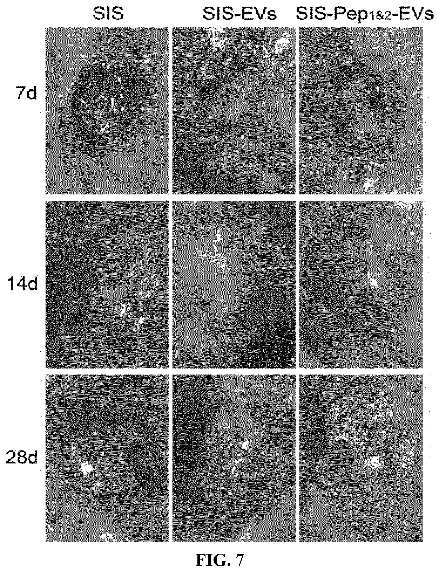

Fig. 7 shows the result of morphological observation after repairing the to-be-repaired part using the modified SIS membrane.

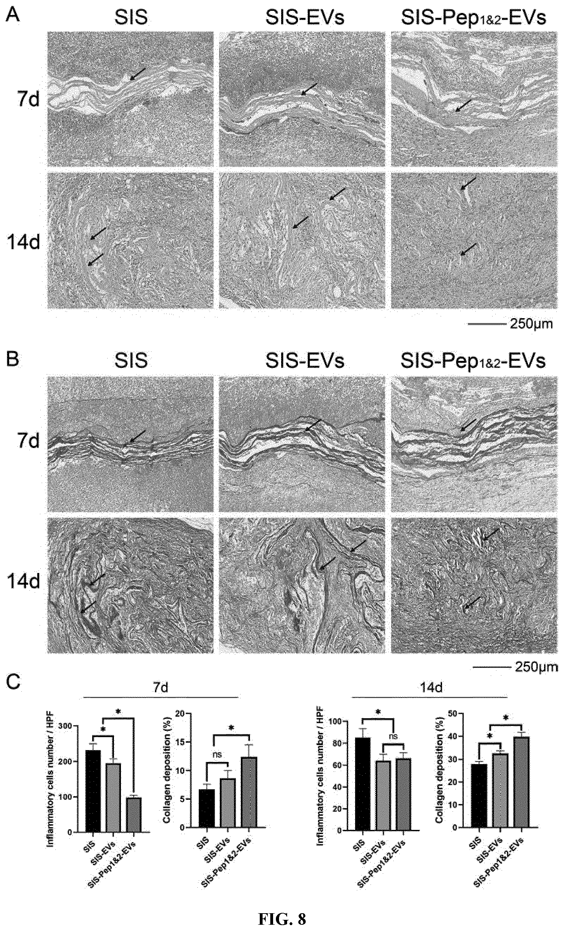

Fig. 8 shows the result of histological analysis. A of Fig. 8 shows the results of HE staining of SIS, SIS-EVs, and SIS-Pep1&2-EVs groups at day 7 and day 14. B of Fig. 8 shows Masson's Trichrome Staining of SIS, SIS-EVs, and SIS-Pep1&2-EVs groups at day 7 and day 14 (the SIS membrane marked with single-direction arrow). C of Fig. 8 shows inflammatory cells and collagen deposition of different groups. All data is expressed as average value±SD, n=6, *P<0.05. One-way ANOVA was used to analyze the result.

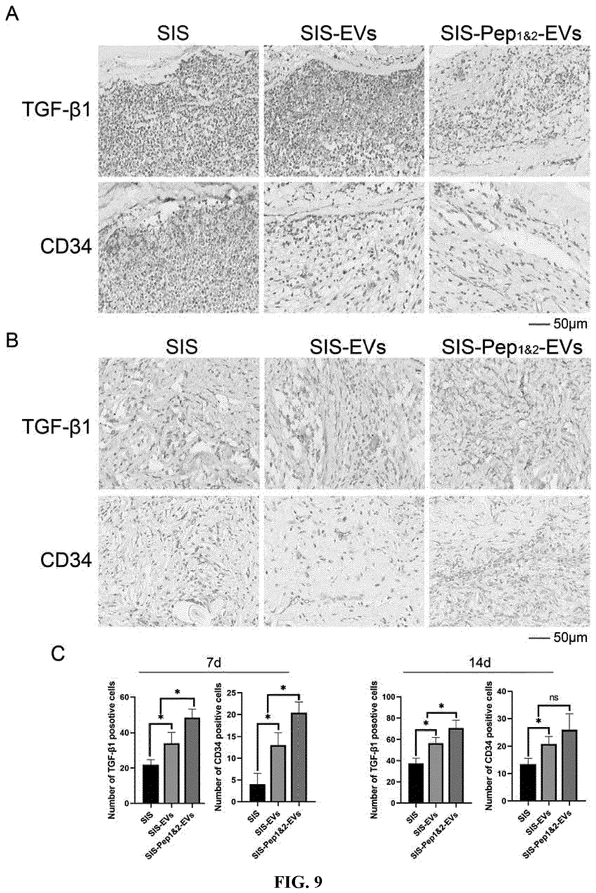

Fig. 9 shows representative immunohistochemical images of TGF-β1 and CD34 in the operating area of the SIS, SIS-EVs, and SIS-Pep1&2-EVs groups at day 7 and day 14. A of Fig. 9 shows the result on the 7th day. B of Fig. 9 shows the result on the 14th day. C of Fig. 9 shows the statistical analysis of TGF-β1 positive cells and CD34 positive cells. All data is expressed as average value± SD, n = 6, *P < 0.05. One-way ANOVA was used to analyze the result.

### DETAILED DESCRIPTION

### Definitions of Terms

When used in combination with the term "including" in the claims and/or specification, the word "a" or "an" may refer to "one", or refer to "one or more", "at least one", or "one or more than one".

As used in the claims and specification, the term "including", "having", "comprising", or "containing" is intended to be inclusive or open-ended, and does not exclude additional or unrecited elements or steps of methods.

Throughout the application document, the term "about" means that a value includes the standard deviation of error caused by the device or method used to measure the value.

It is applicable to the content disclosed herein that the term "or" is defined only as alternatives and "and/or", but the term "or" used herein refers to "and/or" unless otherwise expressly stated to be only alternatives or mutual exclusion between alternatives.

As used herein, the term "mutation of amino acid" or "mutation of nucleotide" includes "substitution, repetition, deletion, or addition of one or more amino acids or nucleotides". In the present disclosure, the term "mutation" refers to a change in a sequence of a nucleotide or in a sequence of an amino acid. In some embodiments, mutation of the present disclosure may be selected from "conservative mutation", "semi-conservative mutation", or "non-conservative mutation". In the present disclosure, the terms "non-conservative mutation" and "semi-conservative mutation" may refer to a mutation that causes loss of function or partial loss of function of proteins; the term "conservative mutation" refers to a mutation that remains normal functions of proteins. A representative example of conservative mutation is conservative substitution.

As used herein, the term "conservative substitution" is usually to exchange one kind of amino acid at one or more sites of a protein. Such a substitution may be conservative. Substitutions taken as conservative substitutions may include, for example, a substitution of Ala with Ser or Thr, a substitution of Arg with Gln, His or Lys, a substitution of Asn with Glu, Gln, Lys, His or Asp, a substitution of Asp with Asn, Glu or Gln, a substitution of Cys with Ser or Ala, a substitution of Gln with Asn, Glu, Lys, His, Asp or Arg, a substitution of Glu with Gly, Asn, Gln, Lys or Asp, a substitution of Gly with Pro, a substitution of His with Asn, Lys, Gln, Arg or Tyr, a substitution of Ile with Leu, Met, Val or Phe, a substitution of Leu with Ile, Met, Val or Phe, a substitution of Lys with Asn, Glu, Gln, His or Arg, a substitution of Met with Ile, Leu, Val or Phe, a substitution of Phe with Trp, Tyr, Met, Ile or Leu, a substitution of Ser with Thr or Ala, a substitution of Thr with Ser or Ala, a substitution of Trp with Phe or Tyr, a substitution of Tyr with His, Phe or Trp, and a substitution of Val with Met, Ile or Leu. In addition, conservative mutations further include mutations that naturally occur as a result of the individual difference, differences in strains and species, etc.

As used herein, the terms "sequence identity" and "identity percentage" refer to the percentage of nucleotides or amino acids that are the same (i.e., identical) between two or more polynucleotides or polypeptides. The sequence identity between two or more polynucleotides or polypeptides may be determined by aligning the nucleotide sequences of polynucleotides or the amino acid sequences of polypeptides and scoring the number of positions at which nucleotide or amino acid residues are identical in the aligned polynucleotides or polypeptides, and comparing the number of these positions with the number of positions at which nucleotide or amino acid residues are different in the aligned polynucleotides or polypeptides. Polynucleotides may differ at one position by, e.g., containing a different nucleotide (i.e., substitution or mutation) or deleting a nucleotide (i.e., insertion or deletion of a nucleotide in one or two polynucleotides). Polypeptides may differ at one position by, e.g., containing a different amino acid (i.e., substitution or mutation) or deleting an amino acid (i.e., insertion or deletion of an amino acid in one or two polypeptides). The sequence identity may be calculated by dividing the number of positions at which nucleotide or amino acid residues are identical by the total number of nucleotide or amino acid residues in the polynucleotides or polypeptides. For example, the identity percentage may be calculated by dividing the number of positions at which nucleotide or amino acid residues are identical by the total number of nucleotide or amino acid residues in the polynucleotides or polypeptides, and multiplying the result by 100.

Illustratively, in the present disclosure, two or more sequences or subsequences, when compared and aligned at maximum correspondence by the sequence comparison algorithm or by the visual inspection measurement, have at least 40%, 50%, 60%, 70%, 80%, 85%, 90%, 91%, 92%, 93%, 94%, 95%, 96%, 97%, 98%, or 99% "sequence identity" or "identity percentage" of nucleotides or amino acid residues. Measurement/calculation of "sequence identity" or "identity percentage" may be based on any suitable region of a sequence, for example a region of a length of at least about 50 residues, a region of a length of at least about 100 residues, a region of a length of at least about 200 residues, a region of a length of at least about 400 residues, or a region of a length of at least about 500 residues. In some embodiments, the sequence is substantially identical over the full length of either or both of the compared biopolymers (i.e., nucleotides or polynucleotides).

As used herein, the term "reverse complementary sequence" refers to a sequence that is reverse to the direction of the sequence of the original polynucleotide and is complementary to the sequence of the original polynucleotide. Illustratively, if the original polynucleotide has a sequence of ACTGAAC, a reverse complementary sequence thereof would be GTTCAGT.

As used herein, the term "polynucleotide" refers to a polymer composed of nucleotides. Polynucleotide can be in the form of an individual fragment or a component of a larger nucleotide sequence structure, and it is derived from a nucleotide sequence separated at least once in quantity or concentration and can be recognized, manipulated, and restored the sequence and its component nucleotide sequences by standard molecular biology methods (such as using a cloning vector). When a nucleotide sequence is represented by a DNA sequence (i.e., A, T, G, C), this also includes an RNA sequence (i.e., A, U, G, C), wherein "U" replaces "T". In other words, "polynucleotide" refers to a nucleotide polymer that has been removed from other nucleotides (an individual fragment or an entire fragment), or may be a constituent part or component of a larger nucleotide structure, such as an expression vector or a polycistronic sequence. The polynucleotide includes DNA, RNA and cDNA sequences. A "recombinant polynucleotide" or "recombinant nucleic acid molecule" pertains to "polynucleotide".

As used herein, the term "recombinant nucleic acid molecule" refers to a polynucleotides having a sequence that is not linked together in nature. A recombinant polynucleotide may be included in a suitable vector which can be used for the transformation to a suitable host cell. Then, the polynucleotide expresses in the recombinant host cell to produce for example a "recombinant polypeptide", "a recombinant protein", "a fusion protein", and the like.

As used herein, the term "linker peptide" and the term "linker" are used interchangeably. It can link the same or different polypeptides or amino acids.

Linker peptide includes flexible linker peptide and rigid linker peptide. In the examples of the present disclosure, flexible linker peptide are selected as the linker peptide. Preferably, (Gly Gly Gly Gly Ser)ₙ is selected as the linker peptide, wherein n is an integer between 1 and 6; or (Gly Gly Gly Gly Thr)ₙ is selected as the linker peptide, wherein n is an integer between 1 and 6. More preferably, the flexible linker peptide of the present disclosure is selected from Gly Gly Gly Gly Ser.

As used herein, "extracellular vesicles (EVs)" refers to vesicles of a dual-membrane structure generated by cells via the paracrine pathway, having diameters ranging from 40nm to 1000nm. Extracellular vesicles widely exist in cell culture supernatants as well as various body fluids (blood, lympha, saliva, urine, semen, milk). They carry various relevant substances derived from cells such as proteins, lipids and the like, and participate in the processes of cell to cell communication, cell migration, angiogenesis, immunoregulation, and the like.

As used herein, the term "high-stringent conditions" means that following the standard DNA blotting procedures, a probe of at least 100 nucleotides in length pre-hybridizes or hybridizes for 12 to 24 hours at 42°C in 5X SSPE (saline sodium phosphate EDTA), 0.3% SDS, 200 µg/ml of sheared and denatured salmon sperm DNA, and 50% formamide. Finally, the vector material is washed three times at 65°C with 2X SSC and 0.2% SDS, each for 15 min.

As used herein, the term "very high-stringent conditions" means that following the standard DNA blotting procedures, a probe of at least 100 nucleotides in length pre-hybridizes or hybridizes for 12 to 24 hours at 42°C in 5X SSPE (saline sodium phosphate EDTA), 0.3% SDS, 200 µg/ml of sheared and denatured salmon sperm DNA, and 50% formamide. Finally, the vector material is washed three times at 70°C with 2X SSC and 0.2% SDS, each for 15 min.

In the present disclosure, unless otherwise emphasized, the term "fusion polypeptide-modified SIS membrane" and the term "fusion polypeptide-mediated SIS membrane" have the same meaning and may be used interchangeably.

Unless otherwise defined or clearly indicated by the context, all technical and scientific terms used in the present disclosure have the same meaning as typically understood by an ordinary skilled person in the field of the present disclosure.

### Technical solution

In the technical solution of the present disclosure, meanings of the SEQ ID NOs used in the nucleotide and amino acid sequence listing in the specification are as below:
SEQ ID NO: 1 sets forth the amino acid sequence of collagen-binding peptide I (CBP1) (LHERHLNNN);
SEQ ID NO: 2 sets forth the amino acid sequence of collagen-binding peptide III (CBP2) (KELNLVY);
SEQ ID NO: 3 sets forth the amino acid sequence of the linker peptide (linker) (GGGGS);
SEQ ID NO: 4 sets forth the amino acid sequence of polypeptide CP05 (CRHSQMTVTSRL);
SEQ ID NO: 5 sets forth the amino acid sequence of polypeptide Pep 1 (LHERHLNNNGGGGSCRHSQMTVTSRL);
SEQ ID NO: 6 sets forth the amino acid sequence of polypeptide Pep2 (KELNLVYGGGGSCRHSQMTVTSRL);
SEQ ID NO: 7 sets forth the amino acid sequence of polypeptide Pep3 (LHERHLNNNCRHSQMTVTSRL);
SEQ ID NO: 8 sets forth the amino acid sequence of polypeptide Pep4 (KELNLVYCRHSQMTVTSRL).
SEQ ID NO: 9 sets forth the forward primer sequence of amplified gene Vgll3;
SEQ ID NO: 10 sets forth the reverse primer sequence of amplified gene Vgll3;
SEQ ID NO: 11 sets forth the forward primer sequence of amplified gene TEAD1;
SEQ ID NO: 12 sets forth the reverse primer sequence of amplified gene TEAD1;
SEQ ID NO: 13 sets forth the forward primer sequence of amplified gene CYR61;
SEQ ID NO: 14 sets forth the reverse primer sequence of amplified gene CYR61;
SEQ ID NO: 15 sets forth the forward primer sequence of amplified gene BIRC5;
SEQ ID NO: 16 sets forth the reverse primer sequence of amplified gene BIRC5;
SEQ ID NO: 17 sets forth the forward primer sequence of amplified gene GAPDH;
SEQ ID NO: 18 sets forth the reverse primer sequence of amplified gene GAPDH.

**Table 1 Sequences of fusion polypeptides shown in the present disclosure**

| SEQ ID NOs. | Compositions of fusion polypeptides | fusion polypeptide sequences |
|---|---|---|
| 5 | CBP1+Linker+CP05 | LHERHLNNNGGGGSCRHSQMTVTSRL |
| 6 | CBP2+Linker+CP05 | KELNLVYGGGGSCRHSQMTVTSRL |
| 7 | CBP1+CP05 | LHERHLNNNCRHSQMTVTSRL |
| 8 | CBP2+CP05 | KELNLVYCRHSQMTVTSRL |

**Table 2 Primer sequences used in the present disclosure**

| Genes | primer sequences (5'-3') |
|---|---|
| Vgll3 | F: AGTTGTGCGGAGGTGATGTAT(SEQ ID NO: 9) |
| | R: CCGGATGATAGCAGGCTGTAG(SEQ ID NO: 10) |
| TEAD1 | F: GAGCGACTCGGCAGATAAGC(SEQ ID NO: 11) |
| | R: CCACACGGCGGATAGATAGC(SEQ ID NO: 12) |
| CYR61 | F: TAAGGTCTGCGCTAAACAACTC(SEQ ID NO: 13) R: CAGATCCCTTTCAGAGCGGT(SEQ ID NO: 14) |
| BIRC5 | F: GAGGCTGGCTTCATCCACTG(SEQ ID NO: 15) |
| | R: ATGCTCCTCTATCGGGTTGTC(SEQ ID NO: 16) |
| GAPDH | F: AGGTCGGTGTGAACGGATTTG(SEQ ID NO: 17) |
| | R: GGGGTCGTTGATGGCAACA(SEQ ID NO: 18) |

Unless otherwise emphasized, the universal testing methods (A) to (T) used in the examples of the present disclosure followed the following steps:

### (A) Design and synthesis of fusion polypeptides:

Polypeptides in the present disclosure were commercially synthesized (Jill Biochemistry Co., Ltd, Shanghai, China) using the Fmoc (9-fluorenylmethyloxycarbonyl) method. The sequences of the polypeptides in the present disclosure are as set forth in SEQ ID NOs: 1-8 in the present disclosure. The purity of each peptide was at least 95%. The molecular architectures of fusion polypeptides used in the present disclosure were predicted and analyzed by the protein analysis website Robetta. The results were displayed by VMD software. Prediction of hydrophilicity was performed with DNAStar software.

### (B) Cell culture

Cell culture: EV-free foetal bovine serum (FBS; Gibco, USA) was obtained by 8 h of centrifugation at 10,000 g. Rat umbilical cord mesenchymal stem cells (rucMSCs) purchased from Qincheng Biotechnology Co., Ltd. (Shanghai, China) were cultured with Dulbecco's modified Eagle's medium (DMEM) (Gibco, USA) supplemented with 10% EV-free FBS and 1% penicillin and streptomycin (Gibco, USA). Cells were cultured under condition of 5% CO₂ at 37 °C. After cells reached 80-85% confluency, the culture medium was replaced and collected and then preserved at -80 °C.

### (C) Isolation and Characterization of EVs

Preserved cell culture medium was centrifuged at 500 g for 20 min, followed by centrifugation at 3,000 g for 20 min and 20,000 g for 1 hour at 4 °C. Then, the supernatant was collected and filtered through a 0.22-µm filter (Millex, USA) to remove cell debris and large cell vesicles. Subsequently, the samples were ultracentrifuged at 100,000 g continuously for 4 hours. The supernatant was discarded, and sterilized PBS was used to resuspend EVs. EVs were obtained by centrifugation at 100,000 g for another 1 hour. Finally, the obtained EVs were dissolved in 50 µl PBS and stored at -80 °C. The total protein concentration of EVs was quantified by the Pierce BCA Protein Assay Kit (Thermo Fisher Scientific, USA).

A nanoparticle size analyzer (Malvern, NS300, UK) was used to measure the size distribution of the EVs obtained. Morphology was visualized using a high-resolution transmission electron microscope (TEM, Hitachi, HT7700, Japan). Finally, EVs were identified by western blot analysis using extracellular vesicle characteristic markers, including Alix, CD63, CD9 and the negative marker cytochrome C, with the corresponding antibodies (Abcam, UK).

### (D) Determination of morphology and size of EVs after binding to fusion polypeptides:

EVs were incubated with Pep1& Pep2 and Pep3& Pep4 at 4 °C for 12 to 16 hours. Then TEM and NTA were used to examine the morphology and particle size.

### (E) Quantitative relationship between peptides and EVs

FITC-labeled Pep1&2 and Pep3&4 were co-incubated with 1mg/mL EVs at 4 °C for 6 h respectively. The unbound peptides were removed by diafiltration tube. Then, supernatant was detected by spectrophotometer. The standard curves were made to determine the concentration of fusion polypeptide. Particle concentration of EVs was obtained by NTA.

### (F) Preparation and Characterization of SIS Membranes Modified by Fusion polypeptide-mediated EVs

The SIS membranes used in the present disclosure were acellular porcine small intestinal submucosa obtained by decellularization and were provided by Bosis Healing Co., Ltd. (Beijing, China). Four fusion polypeptides were dissolved into 200 µM peptide solution respectively. And the same amount of peptide solution was used in the experiment. Pep1 and Pep2 were incubated with EVs, for example at 4 °C for 6 hours, and then transferred to for example 100kD diafiltration tube (Millipore, USA) to remove a small amount of unbound peptides. The operation of Pep3 and Pep4 is the same as above. SIS membranes were cut into small pieces. One set of SIS membranes were only incubated together with EVs, and the other two sets of SIS membranes were incubated with Pep1&2 solution and Pep3&4 solution on a rotating mixer, for example at 4 °C for 12 to 16 hours. SIS membranes with EVs directly loaded were marked as SIS-EVs. SIS membranes combined with Pep1 and Pep2 were labeled SIS-Pep1&2-EVs, and those combined with Pep3 and Pep4 were labeled SIS-Pep3&4-EVs. The microstructure of modified SIS membranes was observed by scanning electron microscopy (SEM, ZEISS, Gemini 300, Germany). Before investigation, SIS membranes of different groups were freeze-dried and sprayed with gold.

Alternatively, SIS-Pep1&2-EVs and SIS-Pep3&4-EVs may be prepared by the following method: Four fusion polypeptides were dissolved into 200 µM peptide solution respectively. And the same amount of peptide solution was used in the experiment. Pep1 and Pep2 were incubated with the cut SIS membranes, for example at 4 °C for 4 to 10 hours, and then transferred to a diafiltration device to remove a small amount of unbound peptides. The operation of Pep3 and Pep4 is the same as above. Subsequently, the incubated SIS membranes were incubated with EVs to obtain SIS-Pep1&2-EVs and SIS-Pep3&4-EVs.

### (G) Assays of the Fusion Polypeptide-mediated EVs Binding to SIS

To detect the binding rate, samples were observed using a confocal laser scanning microscope (Carl Zeiss, LSM900, Germany) to obtain images. LHERHLNNN (collagen binding peptide I) was labeled with fluorescein isothiocyanate (FITC), and KELNLVY (collagen binding peptide III) was labeled with Alexa Fluor 405. EVs were labeled with the DiI.

### (H) Determination of Loading Efficiency of Modified SIS Membranes

200 µ L EVs were incubated with fusion polypeptides to modify SIS membranes of each group, and these samples were incubated on a rotating mixer at 4 °C for 12 to 16 hours. Then rinse twice with PBS. The supernatant of three groups was collected and the content of EVs was detected by NTA to determine the loading efficiency.

### (I) Observation of Release of Fusion Polypeptides-mediated EVs

Fluorescently labeled SIS-EVs, SIS-Pep1&2-EVs and SIS-Pep3&4-EVs were immersed in PBS. After 1, 3 and 5 days, PBS was removed, and the cells were washed gently with PBS. The remaining fusion polypeptides and EVs were observed by CLSM.

### (J) Observation of Uptake of EVs by cells

DiI-labeled EVs and the NIH3T3 cell line were cocultured. Cells were seeded in a 24-well plate (1 × 10⁴ cells/well), and then EVs were added to the culture media and incubated for 12 h, 24 h, and 48 h. Cells were fixed with 4% paraformaldehyde (Solarbio, China) at room temperature for 10 min and permeabilized with 0.5% Triton X-100 for 10 min. Nuclei were stained with DAPI (Thermo Fisher Scientific, USA). Samples were visualized by CLSM.

### (K) Cell Viability Assay

NIH3T3 cells at passages 4-6 were used in the present disclosure. The viability of cells was measured by the CCK-8 assay. Briefly, after sterilization, SIS, SIS-EVs, SIS-Pep1&2-EVs and SIS-Pep3&4-EVs samples were cut into circles with a diameter of 10 mm and placed into a 96-well plate. Subsequently, NIH3T3 cells were seeded in 96-well plates (2000, 100 µL/well). Three replicates were performed for each group. After 1, 2, 3, 4, 5, 6 and 7 days, 10 µL of CCK-8 reagent (Solarbio, China) was added to each well, followed by incubation for 2 h at 37 °C. OD value was calculated at 450 nm absorbance.

### (L) Migration and Cell Morphology Observation

To assess the migration ability of NIH3T3 cells, 1 × 10⁴ cells/well were seeded in the upper chamber of Transwell 24-well plates (Corning, NY, USA) containing 5% EV-free FBS. Then, complete medium containing 10% FBS was added to the lower chamber supplemented with sterilized SIS, SIS-EVs, SIS-Pep1&2-EVs and SIS-Pep3&4-EVs. After 24 h, Transwell chambers were removed and washed twice with PBS. The nonmigrated cells were gently wiped. Then, the cells were fixed with 4% paraformaldehyde for 30 min and stained with 0.1% crystal violet (Sigma-Aldrich, USA) for 30 minutes. Cells were washed 3 times with PBS. Cell migration images were observed with an optical microscope (Olympus, IX71, Japan).

To observe the cytoskeleton of cells in different groups, the membranes were seeded with NIH3T3 (1 × 10⁴ cells/well). After 6 and 24 h of coculture, cells were fixed in 4% paraformaldehyde for 30 min and permeated in 0.5% Triton X-100 for 10 min. Cytoskeleton observation was obtained by Rhodamine B Phalloidin (Cytoskeleton, Inc., USA) staining. The nuclei were stained with DAPI. Cell morphology was observed by CLSM system. Mean cell area and perimeter were quantified using ImageJ software.

### (M) RNA-seq Assay

High-throughput sequencing was performed to detect the altered expression of miRNAs in different samples and enrichment pathways in tissue regeneration. NIH3T3 cells were seeded into 6-well plates with SIS, SIS-EVs, SIS-Pep1&2-EVs and SIS-Pep3&4-EVs. A blank plate without membrane was used as control. After 3 days, cells were scraped and collected and then sent to a company (Novogene Co., Ltd., Beijing, China) to carry out RNA-seq assays.

### (N) Quantitative real-time PCR

Collected cells were cultured in different groups. Total RNA was extracted by TRIzol Reagent (Invitrogen, USA). Synthesis of cDNA was completed using GoScript Reverse Transcription Mix (Promega, USA). Then, qRT-PCR analysis was performed with a LightCycler 480 II System (Roche, LC480II, Switzerland). The mRNA expression changes of Vgll3, TEAD1, CYR61 and BIRC5 were detected quantitatively using the relative standard curve method (2^{-△△CT}). GAPDH was used as control. The PCR primer sequences used in the present disclosure are listed in Table 2.

### (O) Western Blot

Cells in each group were collected and lysed with RIPA (Solarbio, China) to extract protein. Protein from cultured cells was separated by sodium dodecyl sulfate-polyacrylamide gel electrophoresis (SDS-PAGE) and transferred to polyvinylidene fluoride membranes (Immobilon P, Millipore, Billerica, USA). After blocking with 5% bovine serum albumin (BSA, Sigma-Aldrich, USA) for 1 h at room temperature, the membranes were incubated with primary antibodies at 4 °C overnight, followed by maintaining with horseradish peroxidase-conjugated secondary antibodies (Abcam, USA) at 37 °C for 1 h. Primary antibodies included anti YAP 1, antiTEAD1, antiCYR61 and antiGAPDH antibodies (Abcam, USA). The final bands were visualized using ChemiDoc XRS Plus luminescent image analyzers (Bio-Rad, USA) after adding ECL Substrate (Thermo Fisher Scientific, USA). Densitometric quantification of band intensity was carried out with ImageJ software, and the relative expression level of the target protein was normalized to the band intensity of GAPDH.

### (P) Immunofluorescence staining

After 3 days of culture, cells cultured in different groups were fixed with 4% paraformaldehyde, permeabilized with 0.5% Triton X-100 and sealed at room temperature. The cells were incubated overnight at 4 °C with a CYR61 primary antibody at a 1:200 dilution. Then, Alexa Fluor 488-conjugated antirabbit secondary antibody (Abcam, USA) was added to the samples and incubated at room temperature for 1 h. Nuclei were stained with DAPI. CLSM was used to obtain images.

### (Q) Establishment and Repair of Abdominal Wall Defect Model in Rats

Male Sprague-Dawley (SD) rats aged 6 to 8 weeks (200-2250 g) were used in the present disclosure. The animal experiment was approved by the Animal Ethnic Committee of Tianjin Medical University. Rats were divided into three groups: SIS, SIS-EVs and SIS-Pep1&2-EVs groups. Six replicates were used in each group. Rats were anesthetized with isoflurane through inhalation. After sterilization, round full-thickness defects with a diameter of 1 cm were made on the lateral wall of the abdomen. The defects were then repaired by fixing SIS, SIS-EVs or SIS-Pep1&2-EVs. Then, the surgery area was sutured in layers.

### (R) In Vivo Toxicity Study

To determine the toxicity of the modified SIS membranes *in vivo,* blood was collected from the angular vein of rats and samples were placed in EP tubes. The serum was collected by centrifugation at 3000rpm for 15min. The levels of ALT, AST, BUN and Crea were analyzed in the clinical laboratory (Tianjin Medical University Chu Hisen-I Memorial Hospital, Tianjin, China).

### (S) Morphological Observation and Pathological Analysis

For each group, animals were euthanized to collect implants and surrounding tissue at 7, 14, and 30 d after the operation. The morphology of tissues around the operation area was recorded. Samples were embedded in paraffin, dehydrated with ethanol, and then made into sections with a thickness of 5 µm. Hematoxylin and eosin staining (HE) and Masson trichrome staining (Solarbio, China) were carried out to evaluate tissue regeneration. The number of inflammatory cells and the percentage of collagen deposition were calculated by ImageJ.

For immunohistochemical analysis, sections were incubated overnight at 4 °C with primary antibodies against TGF-β1 and CD34 (Abcam, USA) at a 1:200 dilution. Then, the sections were incubated with the secondary antibody for 1 h at room temperature. Subsequently, the sections were incubated with DAB substrate to visualize antibody binding. After hematoxylin counterstaining, the images were observed by the whole landscape imaging quantitative analysis system (PerkinElmer, Vectra Polaris, USA). The number of TGF- β1 positive cells and CD34 positive cells was measured by ImageJ

### (T) Statistical Methods

All results were statistically analyzed by SPSS v.21.0 software. One-way ANOVA was used to compare the mean values of the data. All data are expressed as average value ± standard deviations (SD) of at least three replicate experiments. A value of P < 0.05 was considered statistically significant. (*p < 0.05).

### Examples

Additional objects, features, and advantages of the present disclosure will become apparent from the following detailed description. However, it is appreciated that the detailed description and the specific examples (although representing specific embodiments of the present disclosure) are merely presented for explanatory purposes, because after reading the detailed description, various changes and modifications made within the spirit and the scope of the present disclosure will become apparent to a person skilled in the art.

The experimental techniques and experimental methods used in the examples, unless otherwise specified, are all conventional techniques and methods. For example, experimental methods for which no specific conditions are indicated in the following examples are generally performed according to conventional conditions such as those described by Sambrook et al., Molecular Cloning: A Laboratory Manual (New York: Cold Spring Harbor Laboratory Press, 1989), or those recommended by manufacturers. The materials, reagents, and the like used in the examples, unless otherwise specified, are all commercially available.

### Example 1: Structure and hydrophilicity prediction of polypeptides

The present disclosure investigated four peptides composed of different components, CP05 (CRHSQMTVTSRL) and CBP (LHERHLNNN or KELNLVY), two polypeptides with a linker peptide (GGGGS), named as Pep1 and Pep2, and two polypeptides without a linker peptide (GGGGS), named as Pep3 and Pep4.

Collagen binding peptides LHERHLNNN and KELNLVY were used in the present disclosure because they rely on either nonspecific electrostatic interactions or specific interactions to bind to collagen. Another functional peptide CP05 is responsible for anchoring EVs. Meanwhile, the flexible linker peptide GGGGS was originally chosen to provide structural flexibility and maintain the distance between two functional domains, consequently enhancing the ability of CP05 to capture EVs.

Fig. 1 is a schematic diagram of the SIS membrane modified by fusion polypeptide-mediated extracellular vesicles in the present disclosure.

### Example 2: Characterization of extracellular vesicles

EVs were purified from the culture medium of ucMSCs by ultracentrifugation.

The experiment result was shown in A of Fig. 2. The morphology observed by TEM showed a typical round or cup shape As shown in B and C of Fig. 2, particle size analysis revealed that the diameters of the extracted vehicles ranged from approximately 50 to 200 nm, and the average particle size of EVs was approximately 141.9±1.2 nm, consistent with the TEM results. Some peaks that appeared beyond 200 nm may be caused by the aggregation of EVs or impurities.

Moreover, as a main basis of EV identification, western blot analysis demonstrated that the characteristic surface markers CD63, CD9 and Alix were positive and that Cytochrome C was negative (as shown in D of Fig. 2). These results suggested that EVs were successfully isolated.

To identify the size and morphology of EVs after binding to fusion peptides, TEM and Nanoparticle Tracking Analysis (NTA) were carried out. The results showed that the morphology and particle size of EVs did not change significantly. The quantitative relationship between fusion peptides and EVs was calculated by standard curve, the calculation results indicated that there were about 1.26 × 10⁻¹⁴ M fusion peptide on one EV when co-incubated with Pep1&2 and were about 1.13× 10⁻¹⁴ M fusion peptide when co-incubated with Pep3&4.

### Example 3: Characterization of modified SIS membrane

The concentration screening pre-experiment was carried out. CLSM images showed that Pep1-Pep4 could achieve good binding effect with SIS membrane at 200µM.

The surface morphology of each group was characterized by SEM images. As shown in A of Fig. 3, the unmodified SIS membrane had an asymmetric structure, including a dense layer and a loose layer. Compared with the SIS group, fine particles could be seen on the surfaces of the SIS-EVs, SIS-Pep1&2-EVs and SIS-Pep3&4-EVs groups, and the SIS-Pep1&2-EVs group had the largest number of particles. Meanwhile, the modification of the SIS membrane did not change its intrinsic structure.

As shown in B of Fig. 3, a group of typical CLSM images revealed the binding of peptide-mediated EVs to the loose layer of SIS membrane. Specifically, particles on the SIS-Pep1&2-EVs group were finer and more homogeneously distributed, demonstrating a better targeting efficiency of the SIS-Pep1&2-EVs group. Moreover, compared with SIS-Pep3&4-EVs, there were more DiI-stained EVs on SIS-Pep1&2-EVs, and the SIS-EVs group showed the fewest fluorescent EVs particles, indicating an effective anchoring phenomenon of Pep1&2. Meanwhile, the loading efficiency increased in the order of SIS-EVs, SIS-Pep3&4-EVs and SIS-Pep1&2-EVs, showing a similar trend with CLSM.

Meanwhile, the release of fusion peptides and EVs was also observed by CLSM (as shown in C of Fig. 3). The amino acids Cys and Met are susceptible to oxidation reactions and can be oxidized by oxygen in the air, which affects the stability of peptides. Fusion peptides and EVs on SIS membranes of all groups decreased gradually with time, probably attributed to the degradation and release of these active substances as well as the rupture of collagen fibers to a certain extent. During the whole process, SIS-Pep1&2-EVs showed the most fusion peptides and EVs, indicating a stronger function of Pep1&2. These images indicated that the GGGGS domain might enhance the expression efficiency of collagen-binding peptide and improve biological activity.

Taken together, these results implied that modified SIS membranes were successfully obtained. Researchers have reported that the flexibility of a linker peptide is related to the cooperative function of protein moieties, indicating the importance of linker peptide flexibility in the construction of fusion peptides. More desirable modification effects of Pep 1 and Pep2 on the SIS membrane may be ascribed to the existence of GGGGS, which serves as a passive linker peptide to achieve an appropriate separation of collagen-binding peptide and CP05, resulting in a satisfactory bioactivity of the fusion peptide. Therefore, we can believe that the GGGGS used in this study is sufficient to maintain stability and provides a suitable structure of the fusion peptide. It will be further discussed in the *in vitro* experiments whether the fusion peptide constructed with the collagen-binding peptide and CP05 with GGGGS has a more positive influence on cell behaviors than the fusion peptide constructed with the collagen-binding peptide and CP05 without a linker peptide.

### Example 4: Biocompatibility Assays and Cell Behaviors

The proliferation and migration of fibroblasts are essential steps in tissue regeneration. Accumulating evidence suggests that uMSC-EVs can regulate the tissue repair course by stimulating cell proliferation and migration. Therefore, CCK-8, Transwell and fluorescence assays were used to explore a series of cell activities *in vitro.*

To maintain homeostasis, EVs need to be taken up by surrounding cells. A of Fig. 4 depicts the uptake results of EVs by cells after 12, 24 and 48 hours of culture. The results showed that the fluorescence intensity increased over time and reached a peak at 24 h, demonstrating that EVs could be transported to recipient cells to regulate cell behaviors in prophase of tissue regeneration. At 48 h, the fluorescence intensity decreased slightly, which indicated that EVs were metabolized in cells.

Meanwhile, as shown in B of Fig. 4, the biocompatibility of the modified SIS membrane was assessed by CCK-8 cell counting analysis. In the case of the SIS-Pep1&2-EVs sample, cells showed the highest proliferative activity after 24 hours. Meanwhile, cells in each group proliferated with time and reached the plateau stage at 6 d. The proliferative capability of cells in the SIS-EVs, SIS-Pep1&2-EVs and SIS-Pep3&4-EVs groups was higher than the proliferative capability of the blank group, indicating that modified SIS membranes were noncytotoxic.

Further, in the present disclosure a Transwell trial was carried out to determine the migration ability of NIH3T3 cells incubated with different samples. The experiment results are as shown in C and D of Fig. 4. In the case of SIS-Pep1&2-EVs and SIS-Pep3&4-EVs, the number of cells passing through the membrane was remarkably higher than the number of cells passing through the membrane in the blank plate, SIS and SIS-EVs groups. This finding illustrates that the modified SIS membrane can elicit rapid migration of cells into soft tissue defects and possibly facilitate tissue regeneration in this way. The SIS-Pep1&2-EVs group was more beneficial to cell motility because more cells in the SIS-Pep1&2-EVs group migrated through the membrane than in the SIS-Pep3&4-EVs group.

Furthermore, according to the present disclosure, the morphology of fibroblasts seeded on SIS, SIS-EVs, SIS-Pep1&2-EVs and SIS-Pep3&4-EVs was observed by CLSM. The experiment result is as shown in E of Fig. 4. Confocal images showed that fibroblasts adhered to SIS-Pep1&2-EVs and SIS-Pep3&4-EVs for 6 h exhibited a more stellate-patterned phenotype, whereas the fibroblasts adhered to SIS and SIS-EVs membranes were spherical or narrow. After culture for 24 h, cells in the SIS-Pep1&2-EVs and SIS-Pep3&4-EVs groups had well-stretched actin bundles. Especially on SIS-Pep1&2-EVs, cells exhibited better spreading morphology with the largest mean cell area and perimeter, confirming enhanced cell attachment and cytoskeleton development.

Based on these results, modified SIS membranes possess ideal biocompatibility and noncytotoxicity towards fibroblasts, indicating an important role in cell proliferation, migration and adhesion because of the ECM-mimetic nanofibrous structure and introduction of EVs. Additionally, SIS membranes modified by peptide-mediated EVs exhibit a stronger ability to promote cell activities, implying that peptide-specifically captured EVs have potential applications in material modification. Apparently, in this study, EVs released from modified SIS membranes in the early stage are helpful to regulate intercellular communication, improve the microenvironment, and finally activate a cascade of cell responses. EVs affect cellular and tissue homeostasis in multicellular organisms via the mechanisms of transducing proteins, lipids, nucleic acids and other metabolites.

Accordingly, in this study, EVs possibly control material-to-cell contacts that influence signal molecule transfer and ultimately the microenvironment, thus modulating the fate of receptor cells. These data also identify better biofunctions of the fusion peptide with the linker GGGGS, which could demonstrate that direct fusion between the collagen-binding peptide and CP05 might cause misfolding and have a little effect on its function.

### Example 5: Research of the mechanism of cell behavior

To investigate the regeneration mechanism of SIS-Pep-EVs, RNA-seq and clustering analysis were performed to screen the differential gene expression. The experiment results are as shown in A of Fig. 5 and B of Fig. 5. Compared with the other groups, the SIS-Pep1&2-EVs group showed enrichment in TEAD transcription factors (TEAD1-4), which are crucial for development, proliferation, tissue homeostasis and regeneration. Among these factors, TEAD1 was reported to be associated with cell proliferation, migration and survival. Through further exploration, Vgll3, the known transcription coactivator of TEADs, was also found to be increased in the SIS-Pep1&2-EVs group. Other components served as target genes of TEAD, such as CYR61 and BIRC5, which were upregulated in cells of the SIS-Pep1&2-EVs group. Therefore, we used qRT-PCR and western blot to detect the expression of these factors.

CYR61 is a downstream gene of TEAD critical for cell proliferation and migration. BIRC5, also known as survivin, can inhibit cell apoptosis. The qRT-PCR analysis results as shown in C of Fig. 5 showed that after 3 days, the SIS-Pep1&2-EVs group had the highest mRNAlevels of Vgll3, TEAD1, CYR61 and BIRC5. Consistently, as shown in D and F of Fig. 5, western blot analysis showed that the expression of TEAD1, BIRC5 and CYR61 was the highest in the SIS-Pep1&2-EVs group compared with the other groups after 3 days of incubation. Immunofluorescence of CYR61 as shown in F of Fig. 5 also showed that the fluorescence intensity of the SIS-Pep1&2-EVs group was stronger than that of the other groups, indicating an increased expression level.

The Hippo pathway, which plays an important role in organ size regulation, tissue regeneration and carcinogenesis, has been widely concerned in recent years. The TEAD transcription factor family is an important part of the Hippo pathway. According to above results, Vgll3 and TEAD1 were upregulated in the SIS-Pep1&2-EVs group and then drove the transcription of downstream factors to regulate cell behaviors. Furthermore, the interaction between Vgll3 and TEAD1 is involved in muscle regeneration. Hence, we postulate that the acceleration of regeneration is due partly to Vgll3 and TEAD1 reactions operating with the Hippo pathway by SIS-Pep1&2-EVs. In addition, several studies have manifested that EVs accelerate cutaneous wound healing by transferring miR-21-3p and that the expression of inflammatory genes is reduced when miR-146a is delivered by exogenous EVs in endotoxin injury models. Hence, we speculate that miRNAs contained in EVs may primarily assist in soft tissue regeneration.

### Example 6: In Vivo Wound Healing Assay

To explore the healing efficiency of the modified SIS membrane on the remodelling process, different samples were applied to an abdominal wall defect model in rats.

Considering that the fusion peptides used in this study consisted of collagen binding peptide and CP05, *in vivo* toxicity experiment was conducted to verify whether the modified SIS membrane has any effect on the body. The results as shown in Fig. 6 showed that there was no significant difference in serum analysis of ALT, AST, BUN and Crea among the groups, indicating that the modified SIS membrane was relatively safe without hepatotoxicity and nephrotoxicity.

Morphological observation of the repaired site as shown in Fig. 7 showed that the three groups were not completely healed at 7 d postoperation. The SIS-Pep1&2-EVs group had the smallest defect area, and mild inflammatory reactions could be observed in all groups. At 14 d, obvious connective tissue formation was observed, and the proportion of the defect area decreased gradually. After 30 days, the modified SIS membrane was almost replaced by regenerated tissue without apparent foreign body reaction. In the SIS group, repair of the abdominal wall could also be seen, but the remodelling process was longer than the remodelling process in the SIS-Pep1&2-EVs group.

HE staining of tissues in the surgical area was performed to observe SIS membranes and the reaction with the body after implantation. The result was as shown in Fig. 8. At 7 d, inflammatory cells were found in all groups. Meanwhile, in the SIS-Pep1&2-EVs group, there were fewer inflammatory cells than in the SIS group, and inflammatory cells gradually faded away. At 7 d, all SIS membranes began to biodegrade, and they were covered with more fibrous tissue at 14 d. At 30 d, only a small part of the SIS membrane remained in all groups. Degraded membranes were replaced by regenerative tissue. Thereafter, SIS-Pep1&2-EVs exhibited better biocompatibility because this group presented fewer inflammatory cells in the newly formed tissue, also suggesting that SIS-Pep1&2-EVs may have stronger anti-inflammatory ability, providing a microenvironment in favor of tissue regeneration.

Meanwhile, as shown in Fig. 8, the formation of collagen was observed by Masson's trichrome staining. The collagen content in the three groups increased with time, whereas earlier neovascularization and more collagen deposition were observed in the SIS-Pep1&2-EVs group. More collagen with an arrangement similar to normal tissue could be seen in the SIS-Pep1&2-EVs group without tissue fibrosis at 30 d. This phenomenon indicated that SIS-Pep1&2-EVs can reduce the occurrence of complications, mainly the decrease in abdominal wall compliance.

Immunohistochemical analysis was used to assess the expression of related regulatory factors in tissue repair. CD34 was used to detect the density of neovascularization. As shown in Fig. 9, among the three groups, the CD34 expression level was the highest in the SIS-Pep1&2-EVs group and the lowest in the SIS group at 7 d and 14 d, suggesting that SIS-Pep1&2-EVs was conducive to the formation of blood vessels. Additionally, the SIS-Pep1&2-EVs group had more TGF-β1-positive cells at 7 d and 14 d but fewer TGF-β1-positive cells at 28 d around the defect area. Meanwhile, relatively weak positive expression of TGF-β1 was observed in the SIS group. The expression levels of TGF-β1 and CD34 in the SIS-EVs group were between the expression levels of TGF-β1 and CD34 in the SIS and SIS-Pep1&2-EVs groups. TGF-β1 can regulate inflammatory reactions, angiogenesis and the synthesis of collagen as well as connective tissue. Nevertheless, extended activity will result in tissue fibrosis and scarring. These results suggested that SIS-Pep1&2-EVs may promote TGF-β1 expression in the initial stage of tissue regeneration and reduce expression in the final stage to prevent fibrosis formation.

*In-vivo* experiments showed a fast rate of SIS membrane degradation. Therefore, more active substances must be introduced to provide a better microenvironment for tissue regeneration. These data showed the validity of the modified SIS membrane and that SIS-Pep1&2-EVs contributed to the early phase of remodelling, which was in accordance with *in vitro* experiments. An improved microenvironment for the tissue regeneration and healing process was continuously generated by EVs released from SIS-Pep1&2-EVs. In addition, the therapeutic features of EVs are attributed to their immunomodulatory and immunosuppressive activities. EVs have been reported to suppress the concentration of cytokines, including interleukin 1 beta (IL-1β) and tumor necrosis factor alpha (TNF-α), while increasing transforming growth factor beta (TGF-β) secretion, thereby modulating the immune microenvironment around the wound. Therefore, cross-talk mediated by peptide-mediated EVs between the microenvironment and cells is beneficial to enhance the functional properties of the SIS membrane. Initial healing accelerated by the modified SIS membrane and sustained release of EVs promise to promote tissue reconstruction and restore body function as early as possible.

In conclusion, in the present disclosure, the SIS membrane was treated with EVs mediated by fusion peptides composed of collagen-binding peptide and CP05. SIS-Pep1&2-EVs exhibited an enhanced impact on cell proliferation and migration because of EVs released from the modified SIS membrane, resulting in rapid tissue reconstruction of the abdominal wall. The membrane of the present disclosure is thus a promising material for tissue regeneration.

The afore-described examples of the present disclosure are merely exemplary to clearly explain the present disclosure, and should not be considered to limit the embodiments of the present disclosure. A number of variations and modifications may occur to one skilled in the art based on the foregoing description. It is to be appreciated that the embodiments of the present disclosure are be exhaustively enumerated herein. Various modifications, substitutions, and improvements made without departing from the spirit and the principle of the present disclosure should be included in the scope of protection.

## Claims

1. A composition, comprising a fusion polypeptide-modified SIS membrane and an extracellular vesicle, wherein a sequence of the fusion polypeptide comprises at least one selected from a group consisting of sequences as set forth in the following SEQ ID NOs:
(i) a group consisting of a sequence as set forth in SEQ ID NO: 5, a sequence as set forth in SEQ ID NO: 6, a sequence as set forth in SEQ ID NO: 7, and a sequence as set forth in SEQ ID NO: 8;
(ii) a sequence having one, two, three, four, or five conservative substitutions as compared with the sequence as set forth in (i).

2. The composition according to claim 1, wherein the sequence of the fusion polypeptide is composed of at least one selected from a group consisting of sequences as set forth in the following SEQ ID NOs:
(i) a group consisting of a sequence as set forth in SEQ ID NO: 5, a sequence as set forth in SEQ ID NO: 6, a sequence as set forth in SEQ ID NO: 7, and a sequence as set forth in SEQ ID NO: 8;
(ii) a sequence having one, two, three, four, or five conservative substitutions as compared with the sequence as set forth in (i).

3. The composition according to claim 1 or 2, wherein the sequence of the fusion polypeptide on the fusion polypeptide-modified SIS membrane is set forth in SEQ ID NO: 5 and SEQ ID NO: 6.

4. The composition according to any one of claims 1 to 3, wherein the extracellular vesicle derives from a mesenchymal stem cell; preferably, the extracellular vesicle is an extracellular vesicle derived from an umbilical cord mesenchymal stem cell.

5. The composition according to any one of claims 1 to 4, wherein the composition is obtained by incubating the fusion polypeptide-modified SIS membrane and the extracellular vesicle.

6. A preparation method for the composition according to any one of claims 1 to 5, wherein a method for the modification comprises the following steps (a) to (d), or (i) to (iv):
(a) dissolving the fusion polypeptide in a solvent to obtain a solution containing fusion polypeptides;
(b) mixing and incubating the solution obtained in step (a) and extracellular vesicles, to obtain an incubation solution;
(c) applying the incubation solution obtained in step (b) to a surface of the SIS membrane; preferably, immersing the SIS membrane in the incubation solution obtained in step (b) ;
(d) drying the SIS membrane having the incubation solution on the surface to obtain the composition; or
(i) dissolving the fusion polypeptide in a solvent to obtain a solution containing fusion polypeptides;
(ii) applying the solution obtained in step (i) to a surface of the SIS membrane; preferably, immersing the SIS membrane in the solution obtained in step (i) ;
(iii) applying extracellular vesicles to a surface of the SIS membrane obtained in step (ii) ;
(iv) drying the SIS membrane obtained in step (iii) to obtain the composition.

7. The preparation method according to claim 6, wherein the extracellular vesicle is an extracellular vesicle derived from a mesenchymal stem cell; preferably, the extracellular vesicle is an extracellular vesicle derived from an umbilical cord mesenchymal stem cell.

8. Use of the composition according to any one of claims 1 to 5 or the composition obtained by the preparation method according to claim 6 or 7 in at least one of the following (a) to (c):
(a) as, or for preparation of, a biomaterial for bone generation;
(b) as, or for preparation of, a biomaterial for facilitating tissue healing;
(c) as, or for preparation of, a biomaterial for treating bone defects.

9. A method for treating bone defects or for facilitating tissue healing or bone regeneration, comprising a step of administering the composition according to any one of claims 1 to 5 or the composition obtained by the preparation method according to claim 6 or 7 to a subject.
